Europäisches Patentamt

European Patent Office

Office européen des brevets

(19)

(11) Publication number: **0 139 949**
**B1**

(12) **EUROPEAN PATENT SPECIFICATION**

(45) Date of publication of patent specification: **28.10.87**

(51) Int. Cl.⁴: **A 61 M 1/34, A 61 M 1/36**

(21) Application number: **84109703.3**

(22) Date of filing: **15.08.84**

(54) **Apparatus for purifying blood.**

(30) Priority: **19.08.83 JP 152298/83**

(43) Date of publication of application:
**08.05.85 Bulletin 85/19**

(45) Publication of the grant of the patent:
**28.10.87 Bulletin 87/44**

(84) Designated Contracting States:
**BE CH DE FR GB IT LI NL SE**

(56) References cited:
**DD-A-2 003 118**
**DE-A-3 101 159**
**FR-A-2 510 412**
**US-A-4 243 532**

(73) Proprietor: **KANEGAFUCHI KAGAKU KOGYO
KABUSHIKI KAISHA
2-4 Nakanoshima 3-chome
Kita-ku Osaka-shi Osaka-fu (JP)**

(72) Inventor: **Takata, Satoshi
1-13-704, Yoshida-cho Hyogo-ku
Kobe-shi Hyogo-ken (JP)**

(74) Representative: **Türk, Gille, Hrabal
Bruckner Strasse 20
D-4000 Düsseldorf 13 (DE)**

EP 0 139 949 B1

Courier Press, Leamington Spa, England.

## Description

The present invention relates to an apparatus for purification of blood having a simple structure and capable for separation, purification and mixing of blood; in particular it relates to an apparatus for purification of blood comprising:

a hollow container having a blood flow inlet at one end thereof and a blood flow outlet at the other end thereof;

an impure blood compartment and a purified blood compartment coupled with said inlet and outlet, respectively, so that the blood can flow through them,

a bundle of hollow fibers made of semipermeable membrane connecting said impure blood compartment and said purified blood compartment so that the blood can flow through them, and

a treating compartment, which is provided in the interior of said hollow container, between said impure blood compartment and said purified blood compartment and outside said bundle of hollow fibers, and which is charged with a plasma treating agent.

In recent years, a process for purifying blood, in which blood is separated into plasma and blood cells, the plasma is purified, and then, the purified plasma is mixed with the blood cells again, has been broadly employed. The process has an advantage that various biogenic rejections can be avoided since only objects to be eliminated is taken out from a patient body, and is free from a problem of a loss of the blood cells since only plasma is subjected to a purification.

As a method for separating the blood into plasma and blood cells, there are used, for instance, a centrifugation method, a membrane-employing method using a semipermeable hollow fiber or a porous membrane and the like. For a method for purifying plasma, there is toward a practical use, an adsorbent system employing activated carbon, immunological adsorbent, and the like. Recently, a purifying agent utilizing an enzyme is also proposed.

When the above-mentioned process for purifying blood is carried out, it is necessary to adjust three processes to a most suitable condition, respectively, at all times; i.e. a process of separating plasma from blood, a process of purifying plasma and a process of mixing the purified plasma into the blood having a high concentration of blood cells.

Special care should be paid to the separating process of plasma from blood cells. If an excess pressure or mechanical force is gained to the blood at the time of separation, the blood cells are destroyed to cause a defunctionalization of blood. For preventing such a phenomenon, the membrane-employing method is desirably applied. But also in such a method, it is necessary to keep a trans-membrane pressure within a definite range at all times, and a provision of the condition is complicated and troublesome. That is, to keep a trans-membrane pressure within a definite range, it is necessary to control both a filtration pressure in the blood side and a pressure in the plasma side. Conventionally, two pumps have been employed and operated with suitable care, i.e. a pump for circulating the blood outside a body and a pump for controlling a taking speed of plasma.

Moreover, in a conventional apparatus, a plasma separator and a plasma purifying system must be arranged so that an additional connecting path between them should be provided owing to the above-mentioned requirement for controlling the separating process, and the apparatus becomes complicated.

This is also valid for the apparatus for purification of blood known from FR—A—2510412 comprising:

a hollow container having a blood flow inlet at one end thereof and a blood flow outlet at the other end thereof;

an impure blood compartment and a purified blood compartment coupled with said inlet and outlet, respectively, so that the blood can flow through them,

a bundle of hollow fibers made of semipermeable membrane connecting said impure blood compartment and said purified blood compartment so that the blood can flow through them, and

a treating compartment, which is provided in the interior of said hollow container, between said impure blood compartment and said purified blood compartment and outside said bundle of hollow fibers, and which is charged with a plasma treating agent.

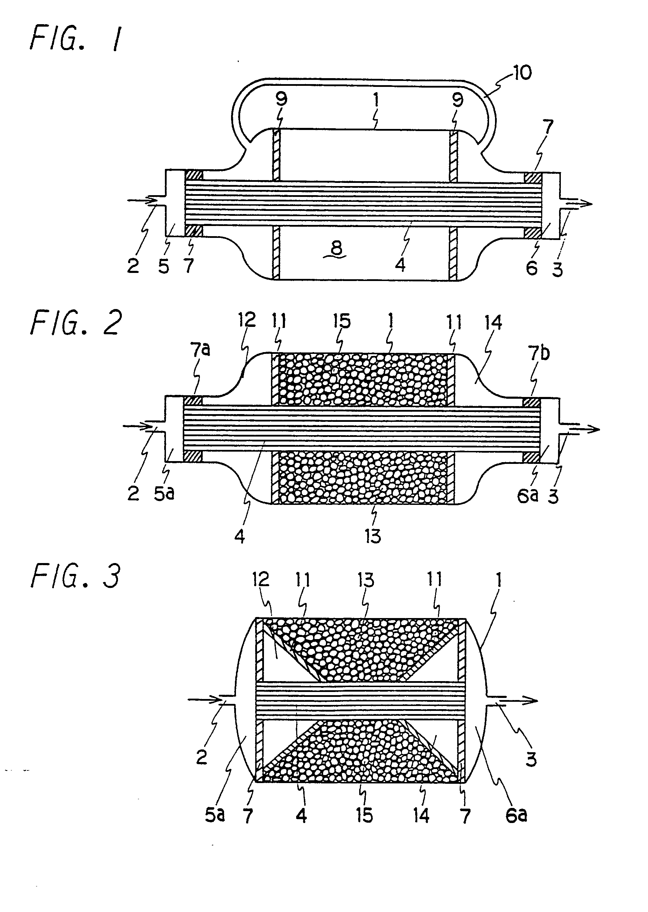

For the purpose of solving many problems as described above, the present inventor has studied earnestly and has found a surprising fact, which is explained hereinafter referring to Fig. 1.

Fig. 1 is a schematic longitudinal sectional view of a plasma separator to explain the principle of the present invention. The plasma separator was made using a bundle of semipermeable hollow fibers as shown in Fig. 1, and a flow of the separated plasma was observed. In Fig. 1, in a container 1, were provided a blood flow inlet 2 at one end thereof and a blood flow outlet 3 at the other end thereof. A bundle of semipermeable hollow fibers 4 was provided in the container 1 to connect two blood compartments 5 and 6, which were provided at the insides of both ends of the container 1, respectively. The both ends of the bundle 4 were fixed to the container 1 with sealants 7 and open to the compartments 5 and 6, respectively.

When blood flowed into the container 1 through the inlet 2 as in the direction of an arrow shown, the blood was separated into plasma and blood rich in blood cells in the bundle 4. The separated plasma flowed into a reservoir 8 surrounded by two blood compartments 5 and 6, the container 1 and the bundle 4. In this context, the reservoir 8 was divided into three spaces with two partition plates 9 and spaces of the both ends were connected with a tube 10, whereby there was found a fact that the separated plasma flowed in the tube 10 at a considerable high speed and its flow was maintained even though a pressure drop within the tube 10 was raised.

The present invention makes use of such a fact, i.e. a reservoir in which the filtrated plasma flows is divided with porous partition plates to form a plasma treating compartment and in the treating compartment is charged a plasma treating agent, whereby the separated plasma can be purified by passing through the treating agent in the treating compartment on account of the flow of the plasma outside of the bundle of hollow fibers, and the purified plasma can be returned to the blood having a high concentration of blood cells.

In accordance with the present invention, there is provided an apparatus for purification of blood comprising:

a hollow container (1) having a blood flow inlet (2) at one end thereof and a blood flow outlet (3) at the other end thereof;

an impure blood compartment (5) and a purified blood compartment (6) coupled with said inlet and outlet, respectively, so that the blood can flow through them,

a bundle of hollow fibers (4) made of semipermeable membrane connecting said impure blood compartment and said purified blood compartment so that the blood can flow through them, and

a treating compartment (13), which is provided in the interior of said hollow container, between said impure blood compartment and said purified blood compartment and outside said bundle of hollow fibers, and which is charged with a plasma treating agent, characterised in that the ends of said treating compartment are defined by porous partition plates (11), whereby plasma can flow into the treating compartment through one of the porous partition plates and out of the treating compartment through the other of the porous partition plates.

Hereinafter, the apparatus for purification of blood of the present invention is explained referring to the attached drawings showing:

Fig. 1 a schematic longitudinal sectional view of a plasma separator to explain the principle of the present invention; and

Fig. 2 and Fig. 3 schematic longitudinal sectional views of the embodiments of the apparatus for purification of blood of the present invention, respectively, wherein the same numbers as in Fig. 1 represent the same members, respectively. Arrow heads in Fig. 2 show a flow of blood.

In Fig. 2, two porous partition plates 11 are provided in the container 1 to divide the reservoir, in which the plasma flows, into an impure plasma compartment 12, a plasma treating compartment 13 and a purified plasma compartment 14. In the plasma treating compartment 13 are charged plasma treating agents 15. The porous partition plate 11 is made of any materials as long as not the treating agent 15 but the plasma can pass through the plate.

Blood is introduced via blood flow inlet 2 into an impure blood compartment 5a by means of an external blood circulation pump (not shown) and is separated into blood having a high concentration of blood cells and plasma during passing through the bundle of hollow fibers 4 due to a flow inlet pressure.

At that time, a pressure difference corresponding to a pressure drop caused by the bundle of hollow fibers arises between the impure blood room 5a and the purified blood compartment 6a and also the blood pressure in the bundle of hollow fibers changes by a gradation that the blood pressure of a nearer point to the inlet 2 is higher.

When the treating agents are not charged in the plasma treating compartment 13, the pressure drop along the plasma flow scarcely arises and the pressure of the plasma is the middle between the pressure in the bundle near the inlet 2 and the pressure in the bundle near the outlet 3, if the plasma is in a closed structure and not taken out of the container. Therefore, in that case, the plasma flows back into the hollow fibers in the vicinity of the downstream side of the fibers because the pressure of the plasma is higher than that in the bundle near the outlet 3. In the conventional manner, the plasma pressure is maintained lower than that in the hollow fibers by taking out the plasma by means of pump.

On the contrary, in the apparatus of the present invention, since the plasma treating compartment 13 is charged with the treating agents, a suitable pressure drop arises in the flow of plasma so that pressure in the purified plasma compartment 14 is maintained larger than that in the hollow fibers of the corresponding portion while pressure in the impure plasma compartment 12 and in the plasma treating compartment 13 are maintained lower than those in the hollow fibers of the corresponding portions, respectively. As the results, the blood flowing in the container is separated into plasma and blood cells in the portions of the bundle of hollow fibers 4 corresponding to the impure plasma compartment 12 and the plasma treating compartment 13 and the separated plasma flows through the plasma treating compartment 13 toward the purified plasma compartment 14, whereby the objects to be removed are removed from the plasma by means of the plasma treating agents 15. The purified plasma returns back into the hollow fibers from the purified plasma compartment 14 and is mixed with the blood having a high concentration of blood cells, sent to the purified blood compartment 6a, and then, flows out from the outlet 3.

Sealant 7a on the side of the impure blood compartment 5a must be made from material through which both the blood cells and the plasma cannot pass, but sealant 7b on the side of the purified blood compartment 6a may be made from the same material as that for the porous partition plates 11.

With respect to the charging of plasma treating agents, it is important to select its kind or charging density so that the pressure relationship between the respective portions becomes as mentioned above due to the pressure drop in the flow of plasma by charging. The plasma treating agent must be selected according to the objects to be removed from the blood. There can be used in the present invention, for instance, activated carbon, alumina, ion exchange resin, synthetic or half-synthetic adsorbent such as adsorbent made of a water-insoluble carrier holding materials having affinity for the objects to be removed, and the like. Moreover, when the plasma treating agent containing immobilized enzymes is employed, the objects to be removed can be removed by a chemical reaction.

As the objects to be removed, there can be exemplified, for instance, waste products, LDL cholesterol, protein bound toxin, various causal objects of diseases related to immunity including an immune complex of an autoantibody, and the like.

The kind or charging density to be selected of the plasma treating agent is also varied according to flow rate of blood, kind and separatability of the semipermeable hollow fiber, permeation rate of plasma, charged amount of the treating agents, and the like, therefore, may be determined by integrating and considering all the requirements and the facts.

A porous hollow fiber usable in the present invention is a porous hollow fiber having a high plasma permeation rate, and in case of using such a fiber, a satisfactorily fast flow rate of plasma can be obtained. A porous hollow fiber having a diameter of pores at the inside surface of 0.01 to 10 $\mu$m, preferably 0.1 to 2 $\mu$m and a permeation rate for pure water not less than 0.015 ml/ $m^2 \cdot min \cdot Pa$ (2 ml/$m^2 \cdot min \cdot mmHg$) preferably of 0.375 ml/$m^2 \cdot min \cdot Pa$ (50 ml/$m^2 \cdot min \cdot mmHg$) is advantageously used. When the diameter of pores is not more than 0.01 $\mu$m, a permeation rate of the objects to be removed is small and a purification efficiency is remarkably lowered. On the contrary, when the diameter of pores is not less than 10 $\mu$m, a blood cell passes through the hollow fiber or blocks the pores of the hollow fiber. When the permeation rate for pure water is not more than 0.015 ml/$m^2 \cdot min \cdot Pa$ (2 ml/ $m^2 \cdot min \cdot mmHg$) too many hollow fibers are necessary in order to effectively purify blood, and as the result, the amount of blood and plasma outside a body is increased during the extracorporeal circulation.

According to the present invention, an inner diameter of the hollow fiber is suitably 250 to 500 $\mu$m and about 2,000 to 4,000 hollow fibers having such an inner diameter are preferably used in the form of a bunch.

The material for the hollow fiber is not limited to any specific materials, as long as it meets the above-mentioned requirements. Typical examples of such a material are, for instance, polysulfone, cellulose acetate, polypropylene, polyethylene, polycarbonate, polyvinylalcohol, ethylene-vinylalcohol copolymer, polyacrylonitrile, polyamide, and the like.

Fig. 3 shows another embodiment of the present invention, wherein the same numbers as in Fig. 2 represent the same members, respectively. An apparatus of Fig. 3 is the same as that of Fig. 2 except that the porous partition plates 11 ure inclined and that the shape of the container 1 is different as shown.

As described above, the apparatus for purification of blood of the present invention is able to proceed the separation process of blood, the purification process of plasma and the mixing process of plasma with blood simultaneously by itself, while originally and conventionally those three processes have been proceeded in the separated three devices, respectively, and therefore, the pump for circulating plasma and the plasma path are eliminated from the apparatus. Further, in the present invention, the purified blood can be obtained from the blood flow outlet by using only the pump for circulating blood outside a body, and moreover, complicated and troublesome operations required for controlling pressure during separation of blood can be largely reduced.

The present invention is more specifically described and explained by means of the following Example.

Example

Using the apparatus as shown in Fig. 2, the waste products were removed from fresh cow blood.

In a container having a length of 23 cm and an outer diameter of 50 mm, was provided a bundle of hollow fibers consisting of 2,700 porous hollow fibers of polysulfone having an outer diameter of 400 $\mu$m, an inner diameter of 300 $\mu$m, a diameter of pores at the inside surface of 0.2 $\mu$m, a diameter of pores at the outside surface of 0.8 $\mu$m and a permeation rate for pure water of 3.75 ml/ $m^2 \cdot min \cdot Pa$ (500 ml/$m^2 \cdot min \cdot mmHg$) and the both ends of a bundle were fixed to the container with sealants of polyurethane, respectively. Two of the porous partition plates made of polycarbonate having a diameter of pores of 1 $\mu$m were provided in the container at the positions of 50 mm from the ends of the bundle of hollow fibers, respectively. 2,000 g of activated carbon having average particle size of 0.8 mm were charged between the porous partition plates.

Through the thus constructed apparatus, 4 l of fresh cow blood was circulated and passed at a flowing rate of 100 ml/min. The blood which was obtained from the blood flow outlet had concentrations of uric acid and creatinine decreased by 58% and 63% in comparison with those in the blood at the blood flow inlet, respectively.

**Claims**

1. An apparatus for purification of blood comprising:

a hollow container (1) having a blood flow inlet (2) at one end thereof and a blood flow outlet (3) at the other end thereof;

an impure blood compartment (5) and a purified blood compartment (6) coupled with said inlet and outlet, respectively, so that the blood can flow through them,

a bundle of hollow fibers (4) made of semipermeable membrane connecting said impure blood compartment and said purified blood compartment so that the blood can flow through them, and

a treating compartment (13), which is provided in the interior of said hollow container, between said impure blood compartment and said purified blood compartment and outside said bundle of hollow fibers, and which is charged with a plasma treating agent, characterised in that the ends of said treating compartment are defined by porous partition plates (11), whereby plasma can flow into the treating compartment through one of the porous partition plates and out of the treating compartment through the other of the porous partition plates.

2. The apparatus of Claim 1, wherein the diameter of the pores at the inside surface of said hollow fibers (4) is 0.01 to 10 µm.

3. The apparatus of Claim 1, wherein said hollow fiber (4) has a permeation rate for pure water of not less than 0.015 ml/m² · min · Pa (2 ml/m² · min · mmHg).

**Patentansprüche**

1. Ein Apparat zur Blutreinigung, der folgendes enthält:

— einen hohlen Behälter (1), der an seinem einen Ende einen Bluteinlass (2) und an seinem anderen Ende einen Blutauslass (3) aufweist;
— ein Abteil (5) für verunreinigtes Blut und ein Abteil für gereinigtes Blut, die jeweils an den Bluteinlass und an den Blutauslass angeschlossen sind, so dass das Blut sie durchqueren kann;
— ein Bündel hohler Fasern (4) aus einer halbdurchlässigen Membrane, das jeweils an das Abteil für verunreinigtes Blut und an das Abteil für gereinigtes Blut angeschlossen ist, so dass das Blut dazwischen fliessen kann;
— ein Behandlungsabteil (13), das innerhalb des hohlen Behälters zwischen dem Abteil für verunreinigtes Blut und dem Abteil für gereinigtes Blut und ausserhalb des Bündels hohler Fasern angeordnet ist und das mit einer Behandlungssubstanz für Plasma befüllt ist,

dadurch gekennzeichnet, dass die Enden dieses Behandlungsabteils durch poröse Trennplatten (11) gebildet werden, so dass das Plasma durch eine dieser porösen Trennplatten in das Behandlungsabteil fliessen kann und durch die andere der porösen Trennplatten aus dem Behandlungsabteil herausflissen kann.

2. Apparat nach Anspruch 1, dadurch gekennzeichnet, dass der Porendurchmesser an der Innenfläche der hohlen Fasern (4) zwischen 0,01 und 10 µ beträgt.

3. Apparat nach Anspruch 1, dadurch gekennzeichnet, dass die hohlen Fasern (4) eine Durchflussrate für reines Wasser von mindestens 0,015 ml/m² · min · Pa (2 ml/m² · min · mm Hg) aufweisen.

**Revendications**

1. Appareil pour l'épuration du sang, comprenant:

un récipient creux (11) possédant une entrée de courant sanguin (2) à une de ses extrémités et une sortie de courant sanguin (3) à son autre extrémité;

un compartiment à sang impur (5) et un compartiment à sang épuré (6) qui sont respectivement raccordés à ladite entrée et à ladite sortie, de manière que le sang puisse les traverser;

un faisceau de fibres creuses (4) faites d'une membrane semi-perméable et qui relient ledit compartiment à sang impur audit compartiment à sant épuré de manière que le sang puisse y circuler; et

un compartiment de traitement (13) qui est prévu dans le volume intérieur dudit récipient creux, entre ledit compartiment à sang impur et ledit compartiment à sang épuré, et à l'extérieur dudit faisceau de fibres creuses, et qui est chargé d'un agent de traitement du plasma,

caractèrisé en ce que les extrémités dudit compartiment de traitement sont définies par des plaques séparatrices poreuses (11), de sorte que le plasma peut pénétrer dans le compartiment de traitement à travers l'une des plaques séparatrices poreuses et sortir du compartiment de traitement à travers l'autre des plaques séparatrices poreuses.

2. Appareil selon la revendication 1, dans lequel le diamètre des pores est de 0,01 à 10 µm au niveau de la surface intérieure desdites fibres creuses (4).

3. Appareil selon la revendication 1, dans lequel ladite fibre creuse (4) possède un taux de perméabilité par rapport à l'eau pure non inférieur à 0,015 ml/m² · mn · Pa (2 ml/m² · mn · mm/Hg).

FIG. 1

FIG. 2

FIG. 3